# EUROPEAN PATENT APPLICATION

(11) **EP 4 725 511 A1**
(43) Date of publication of application: **15.04.2026**
(21) Application number: 24205302.3
(22) Date of filing: 08.10.2024
(51) Int. Cl.: A61L 9/14, D06F 57/00, D06F 57/12, D06F 58/44, B05B 16/00, D06F 58/20, D06B 5/00

(54) **SCENTING MACHINE FOR FABRICS AND OBJECTS**

(71) Applicant: Globalservicemanagement Italia - S.r.l., 00191 ROMA (IT)
(72) Inventor: LUCIANI, Lucio, 00012 GUIDONIA MONTECELIO (RM) (IT)
(74) Representative: Cutropia, Gianluigi

(57) **Abstract**

A scenting machine (100) for objects (P) to be perfumed, comprises a frame (1), a misting chamber (15), a loading door (3), spary nozzles (4), a container (5) filled with a perfumed liquid (50), a hydraulic conduit (51) connecting the container (5) to the spary nozzles (4), a pump (52) for feeding the perfumed liquid to the spary nozzles (4), a flywheel (V) mounted rotatably in the misting chamber (15), an electric motor (6) which rotates said flywheel (V), and gripping means (7) connected to said flywheel (V) and suitable for clamping the objects (P) to be perfumed.

## Description

The present invention refers to a scenting machine for fabrics and medium-sized objects. Fabrics are understood to include clothing, linen, sheets, sponges in general, blankets, carpets and the like, and objects are understood to include shoes, bags, accessories and, in general, porous objects.

There are known industrial laundries, in outsourcing, that deal with treating, with particular treatment cycles (washing, drying and ironing), the linen that hotels and in general accommodation facilities use to dress their rooms. These treatment cycles, in order to ensure the priority sanitization of the fabrics, are carried out at very high temperatures, generally 80-90°C. Therefore, the addition of scented essences during the entire treatment cycle of an industrial laundry would be totally ineffective since, at such high temperatures, the scented essences evaporate and do not adhere to the fabric.

However, hotel guests, in a trend that sees them increasingly demanding and attentive to service, expect that the linen they find in the hotel room (sheets and sponges), in addition to being clean and ironed, is also scented. In this case, the hotels themselves are forced to purchase scented substances, generally spray-type, which are sprayed manually on the fold of the sheet and on the bath sponges, supplied clean by the external laundry. This operation is carried out by the maids when they make up the rooms.

Obviously, such a system of manual perfuming of fabrics is not very effective (because it disappears in a few hours) and not very efficient because, in addition to being very expensive, it requires a lot of time and manpower, and is left to the expertise of the housekeeping employee.

KR2017011051A discloses a fabric scenting machine according to the preamble of claim 1.

US10294604B3 discloses a drying apparatus and an additive distribution unit configured to deliver a mist of additive to the drum.

US2016/037865A1 discloses a device for applying an impregnating agent to the surfaces of footwear.

US2006/101867 discloses an apparatus for processing garments that includes a system for introducing air and water.

The purpose of the present invention is to eliminate the drawbacks of the prior art, providing a fabric and object scenting machine that is efficient and capable of perfuming the product in a long-lasting manner, directly at hotels and accommodation facilities in general, or at nursing homes and healthcare facilities, or even at laundries themselves.

Another object of the present invention is to provide such a scenting machine that is versatile and suitable for treating a plurality of products simultaneously, with an automated treatment cycle.

Yet another object of the present invention is to provide such a scenting machine that is economical and simple to construct and use, even when faced with many packages of laundry to be perfumed in sequence.

These objects are achieved in accordance with the invention with the features of the annexed independent claim 1.

Advantageous embodiments of the invention appear from the dependent claims.

The scenting machine according to the invention is defined by claim 1.

The scenting machine is intended for perfuming linen, blankets, duvets, carpets, curtains and anything that the loading area of the machine can accommodate. The machine is intended primarily for hotel facilities, but can also be used in laundries or for private use.

This machine is characterized by a clamping of the products and their rotation which causes an expansion of the products by centrifugal force and a penetration of the nebulized fragrance into the innermost areas of the objects to be perfumed. This machine is also characterized by a particular clamping system which clamps the objects placed in the misting chamber of the machine.

Further features of the invention will appear clearer from the detailed description that follows, referring to a purely exemplary and therefore non-limiting embodiment thereof, illustrated in the attached drawings, in which:
Fig. 1 is a front view of the scenting machine according to the invention;
Fig. 2 is a perspective view of an upper part of the machine of Fig. 1, with the loading door open;
Fig. 2A is a cross-sectional view of a spray nozzle, taken along the section plane A-A of Fig. 2;
Fig. 3 is a rear view of the scenting machine according to the invention;
Fig. 4 is a perspective view illustrating the rotating flywheel of the scenting machine according to the invention;
Fig. 5 is a front view of the front disk of the flywheel of Fig. 4;
Fig. 6 is a rear perspective view of the flywheel, taken from another angle than Fig. 4;
Fig. 7 is a front perspective view illustrating a plurality of towels clamped between the clamps of the scenting machine according to the invention;
Fig. 8 is a front perspective view like Fig. 7 but during rotation of the flywheel and atomization by the spray nozzles;
Fig. 9 is a photograph of the machine according to the invention, taken from one side of the machine, during operation of the machine; and
Fig. 10 is a block diagram illustrating the operation of the scenting machine according to the invention.

With the aid of the Figures, the scenting machine according to the invention is described, indicated overall with the reference number 100.

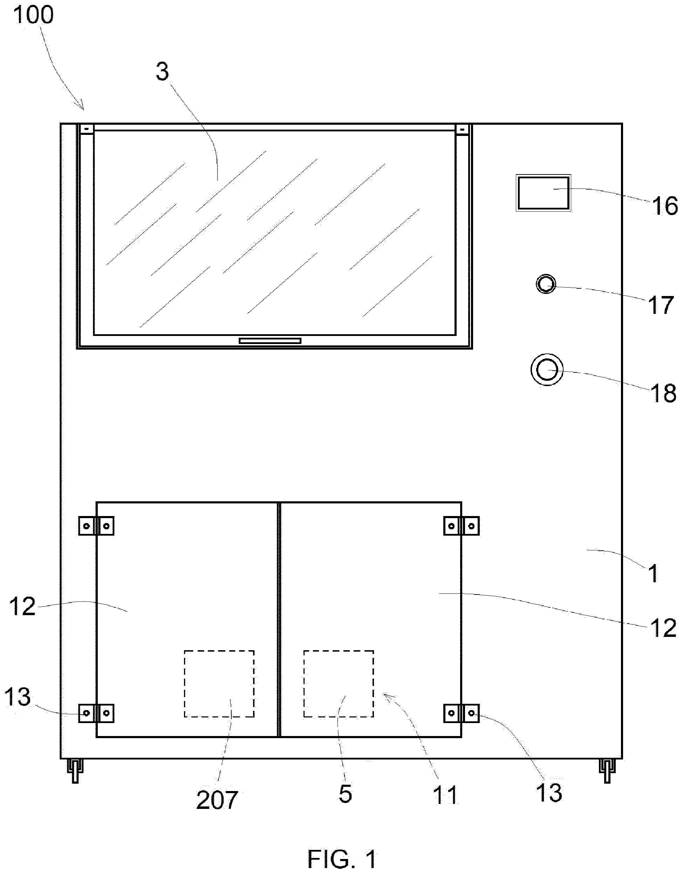

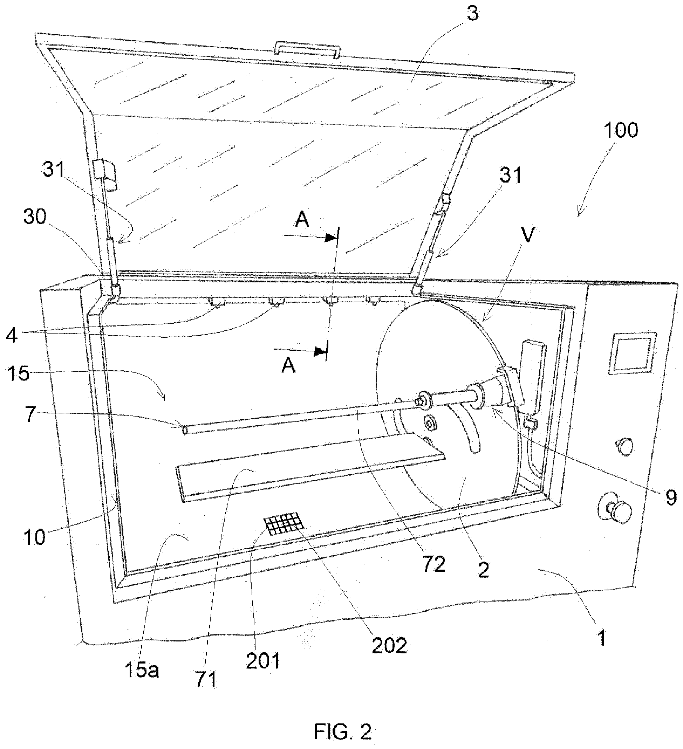

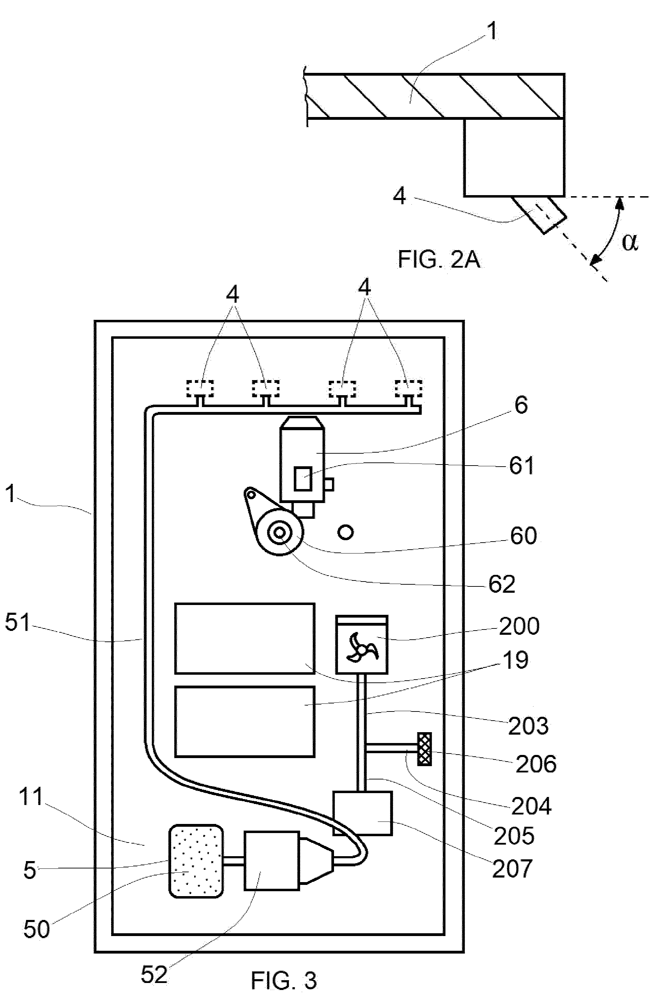

For now, with reference to Figs. 1 and 2, the scenting machine (100) comprises a frame (1) in the shape of a parallelepiped with dimensions of 1250 x 750 x 1520 mm.

A misting chamber (15) is defined within the frame (1). The misting chamber (15) is arranged in an upper part of the frame (1) and is open frontally and/or above, via a loading opening (10).

The misting chamber (15) occupies approximately half the height of the frame (1). Therefore, the misting chamber (1) has a parallelepiped shape with dimensions of 1250 x 750 x 750 mm.

The loading opening (10) is closed by means of a loading door (3). The loading door (3) is hinged to an upper part of the frame (1) by means of hinges (30). The movement of the loading door (3) is cushioned by means of shock absorbers (31) such as gas cylinder-piston groups.

If the misting chamber (15) is open at the front and top, as shown in Fig. 2, the loading door (3) has a substantially "L" shape in cross-section.

Advantageously, the loading door (3) is made of transparent material such as glass or transparent plastic, so that the user can see the contents of the misting chamber (15).

A plurality of spary nozzles (4) are mounted in an upper and rear part of the frame (1) and oriented downwards and towards the centre of the misting chamber (15). For example, four spary nozzles are provided arranged in a row along a longitudinal direction of the machine and with nozzles equidistant from each other.

Referring to Fig. 2A, each spray nozzle is oriented downwards at an angle (α) of 30-60°, preferably 45°, with respect to a horizontal plane. Each spray nozzle (4) has a hole (40) of 0.1 - 0.2 mm, preferably 0.15 mm, which allows the formation of a mist with particles smaller than 0.5µm.

With reference to Figs. 1 and 3, in the lower part of the frame (1) there is a storage compartment (11) which houses a container (5) intended to be filled with a scented liquid (50). The scented liquid (50) may comprise essential oils diluted in alcohol.

The storage compartment (11) is open at the front and may be closed by one or more doors (12) hinged by means of hinges (13) to the frame (1).

The container (5) is connected by means of a hydraulic duct (51) to the spary nozzles (4) to supply the scented liquid (50) to the nozzles. A pump (52) is arranged in the hydraulic duct (51) to pump the scented liquid (50) under pressure to the spary nozzles (4). The pump (52) is a high pressure pump configured to work with a pressure greater than 50 bar, preferably equal to 100 bar. The pump (52) has a flow rate greater than 0.5 l/m permissibly equal to 1.0 I/m.

With reference to Fig. 3, the scenting machine (100) comprises a suction fan (200) to suck air into the misting chamber (15). For this purpose, as shown in Fig. 2, the misting chamber (15) has a base (15a) with an opening (201) in which a grid (202) is arranged. The suction fan (200) is arranged below the base (15a) of the misting chamber to suck air from the opening (201) of the base of the misting chamber.

The suction fan (200) is connected to an exhaust duct (203) having a first outlet (204) and a second outlet (205). The first outlet (204) of the exhaust duct discharges to the outside and is used for the discharge of a gaseous component to the outside. The second outlet (205) of the exhaust duct is used to discharge a liquid/solid component into a discharge container (207) located in the storage compartment (11) near the container (5) containing the perfumed liquid.

The first outlet (204) of the exhaust duct is connected to a filter (206) which retains any liquid and solid impurities, preventing them from being discharged outside.

With reference to Fig. 2, the scenting machine (100) comprises a flywheel (V) mounted rotatably in the misting chamber (15) so as to rotate around a longitudinal rotation axis (X).

With reference to Fig. 3, the flywheel (V) is rotated by an electric motor (6), possibly by means of a gearmotor (60). The electric motor (6) can be equipped with an inverter (61).

Returning to Fig. 2, the flywheel (V) is arranged on one side of the atomizing chamber. On the flywheel (V) are mounted gripping means (7) that protrude into the atomizing chamber (15) in the direction of the rotation axis (X). The gripping means (7) are designed to grip and hold objects still during the rotation of the flywheel (V).

Drive means (9) actuate the gripping means (7) to clamp the objects between the gripping means or disengage the objects from the gripping means.

Preferably the gripping means (7) comprise a gripper comprising a first jaw (71) and a second jaw (72) that clamp the objects to be perfumed together.

The first jaw (71) has the shape of a plate that extends from the flywheel (V) into the atomizing chamber (15), in a direction parallel to the rotation axis (X).

The second jaw (72) has the shape of a rod that extends from the flywheel (V) into the atomizing chamber (15), in a direction parallel to the rotation axis (X) and in a diametrically opposite position to the first jaw (71).

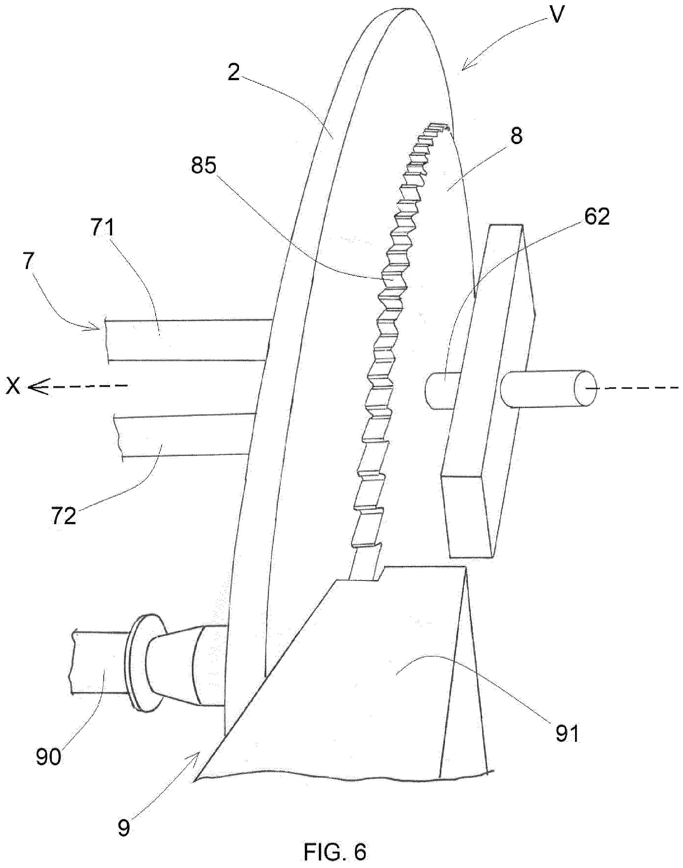

With reference to Fig. 6, the flywheel (V) comprises a front disk (2) facing the atomizing chamber and a rear disk (8) arranged coaxially behind the front disk. The rear disk (8) is connected to a shaft (62) rotated by the electric motor (6) around the rotation axis (X). Then the rear disk (8) rotates around the rotation axis (X) via the electric motor (6).

The front disk (2) is rotatable with respect to the rear disk (8). The drive means (9) enable and disable the rotation of the front disk (2) with respect to the rear disk (8).

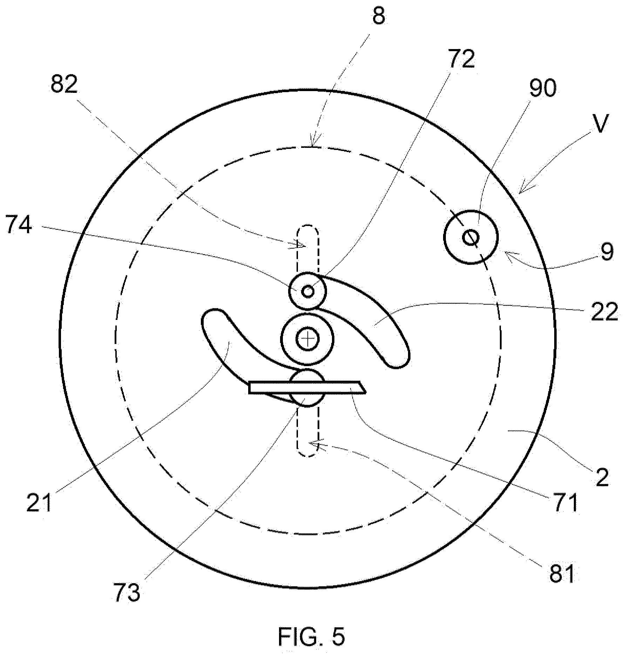

With reference to Fig. 5, in the rear disc (8) a first straight slot (81) and a second straight slot (82) are obtained, arranged in diametrically opposite positions with respect to the centre of the rear disc.

In the front disc (2) a first curved slot (21), comma-shaped, and a second curved slot (82) comma-shaped, are obtained, arranged on either side with respect to the centre of the front disc which corresponds to the centre of the rear disc.

The first curved slot (21) of the front disc communicates with the first straight slot (81) of the rear disc. The second curved slot (22) of the front disc communicates with the second straight slot (82) of the rear disc.

The first jaw (71) of the gripping means (7) is mounted on a pin-shaped follower (73) that slides within the first curved slot (21) of the front disc and the first straight slot (81) of the rear disc.

The second jaw (72) of the gripping means (7) is mounted on a pin-shaped follower (74) that slides within the second curved slot (22) of the front disc and the second straight slot (82) of the rear disc.

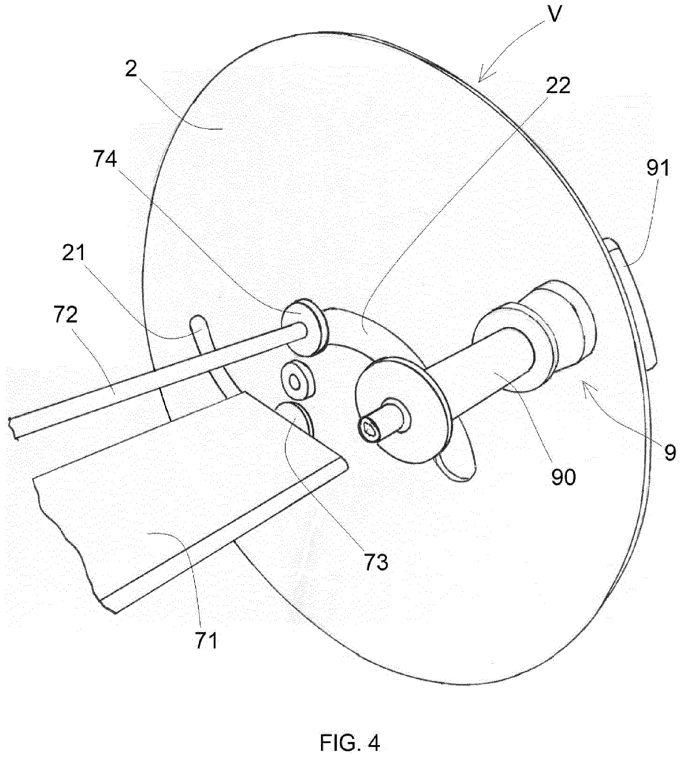

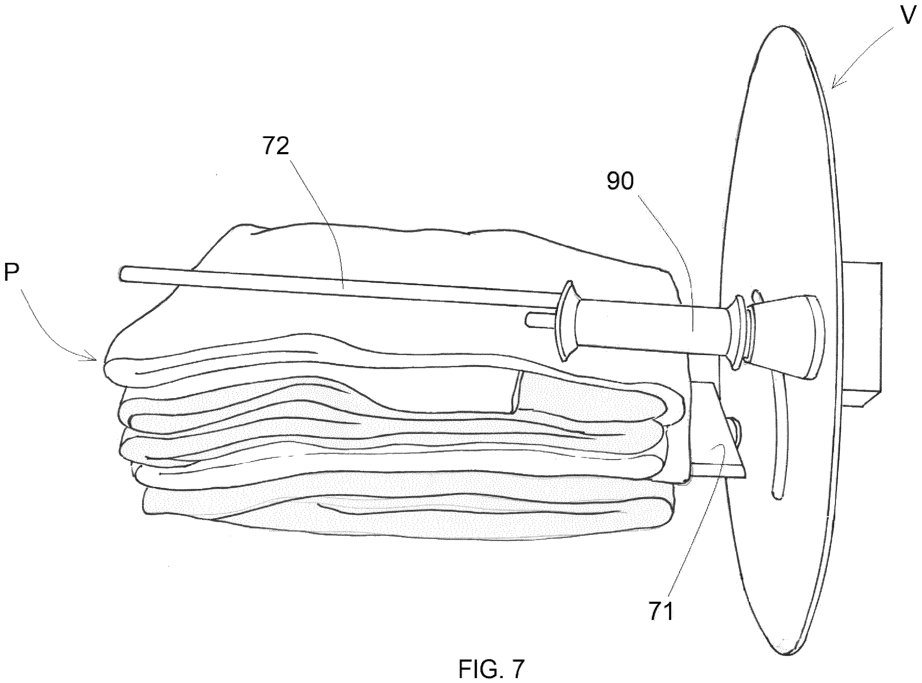

With reference to Figs. 4 and 7, the drive means (9) comprises a handle (90) that protrudes from the front disc (2) in a peripheral position so that it can be gripped by a user, so that the user can rotate the front disc (2) relative to the rear disc (8).

The drive means (9) further comprises a locking device (91) mounted in the front disc (2) protruding rearward from the front disc (2) to lock the rear disc (8) to the front disc.

Advantageously the rear disc (8) may be a toothed wheel having a toothing (85) and the locking device (91) may be a ratchet that engages with the toothing (85) of the toothed wheel to lock the front disc to the rear disc.

By operating the handle (90) by the user, the front disc (2) can move from a first opening position in which the two jaws (71, 72) are distal to a closing position in which the two jaws (71, 72) are proximal to clamp objects between them. When the front disc (2) is in the closed position, the locking device (91) locks the rear disc (8) so that the rear disc can rotate in solidarity with the front disc.

When the front disc (2) is in the open position, the followers (73, 74) of the jaws (71, 72) are located in the straight slots (81, 82) of the rear disc at the ends of the slots distal to the centre of the rear disc.

By rotating the front disc (2), by manually operating the handle (90), the curved slots (21, 22) of the front disc act as cams and push the followers (73, 74) of the jaws (71, 72) onto the straight slots (81, 82) of the rear disc, making them approach the centre of the rear disc, until the front disc (2) reaches the closed position in which the followers (73, 74) of the jaws (71, 72) are located in the straight slots (81, 82) of the rear disc at the ends of the slots proximal to the centre of the rear disc.

With reference to Fig. 1, a touch screen display (16) is mounted in the frame (1) to modify the parameters of the perfuming cycle, a start button (17) to start the perfuming cycle and a safety button (18) that interrupts the operation of the scenting machine (100).

With reference to Fig. 3, a PLC (19) is mounted in a rear part of the frame (1).

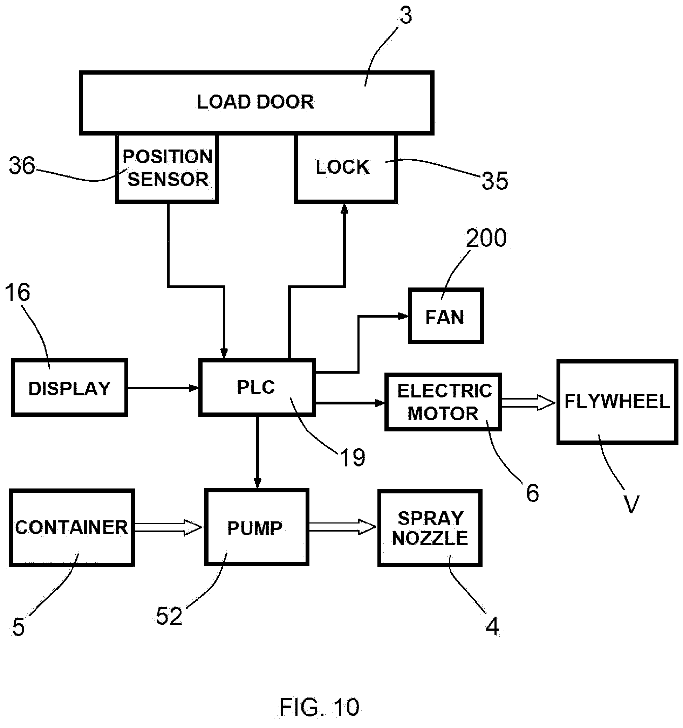

With reference to Fig. 10, the PLC (19) controls the perfuming cycle in its fundamental parameters:
- operating time of the pump (52) which determines the nebulization time of the perfumed liquid by the spary nozzles (4);
- speed (number of revolutions per minute) of the motor (6); and
- operating time of the fan (200) which determines the suction time of the residues of the nebulized fragrance left in the misting chamber (15) at the end of the nebulization cycle.

These parameters can be modified via the display (16) located on the front of the machine.

The scenting machine (100) also includes an electric lock (35) of the loading door (3) controlled by the PLC. When the loading door (3) is closed, the electric lock (35) is blocked and cannot be opened during the perfuming cycle. When the perfuming and suction cycle ends, the PLC (19) unlocks the electric lock (35) so that the user can open the loading door (3).

The scenting machine (100) can also include a position sensor (36) to detect when the loading door (3) is closed and send this information to the PLC (19).

An electric power supply powers the motor (6), the pump (52), the fan (200) and the PLC (19). This electric power supply is connected to an electric cable intended to be connected to the electrical network (220 Vac).

With reference to Fig. 7, the situation is illustrated in which the product (P) to be perfumed are, for example, towels arranged on the first jaw (71) which has a plate shape and clamped between the first jaw (71) and the second jaw (72).

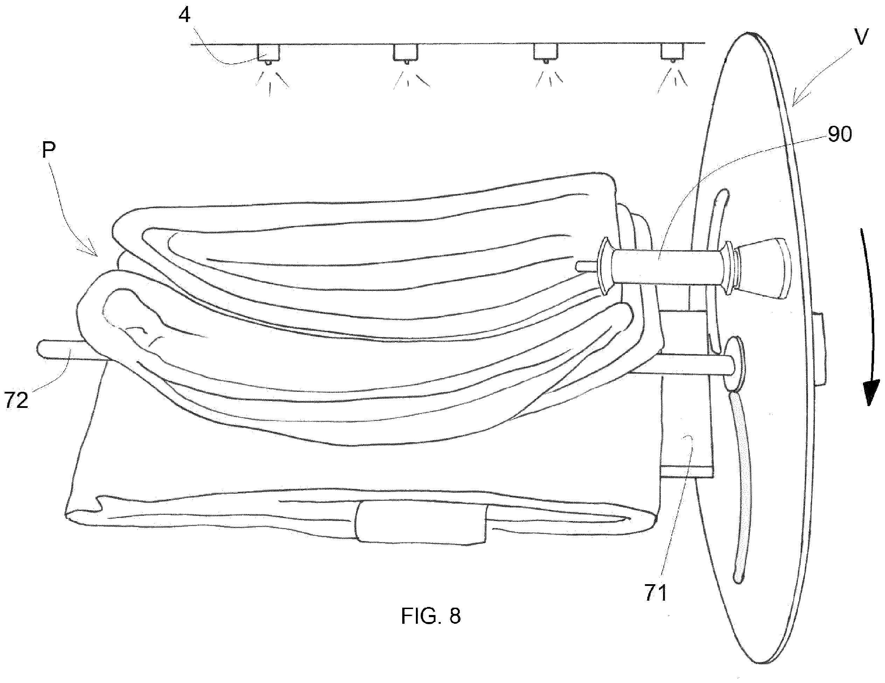

With reference to Fig. 8, during the rotation of the flywheel (V), the product (P) clamped by the gripping means (7) rotates together with the gripping means. Therefore, during the rotation, the product (P) is subject to a centrifugal force that tends to expand it. In this way, the nebulized perfume released by the nebulizing nozzles (4) tends to penetrate intimately into the product (P), allowing for a uniform and long-lasting fragrance.

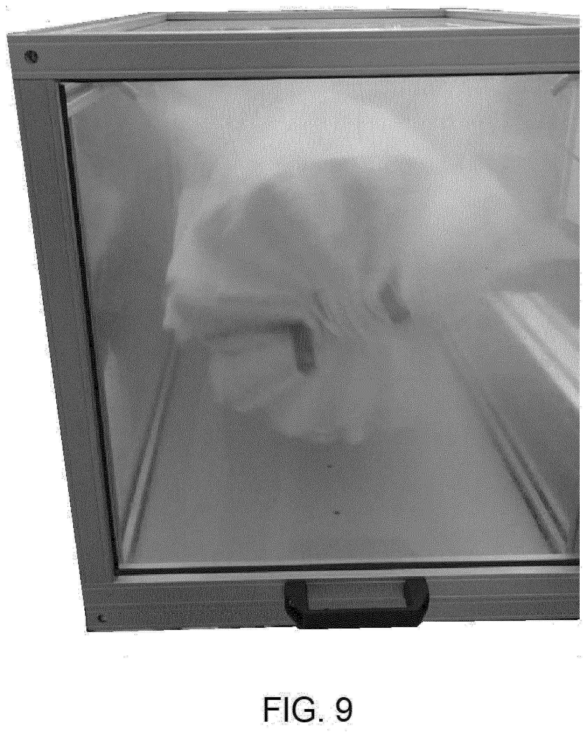

Fig. 9 is a photograph of the scenting machine (100) during the rotation of the gripping means (7) that hold a plurality of towels. As can be seen from this photograph, the towels are subject to centrifugal force, therefore they expand, creating gaps in which the nebulized perfume released by the nebulizing nozzles (4) can penetrate.

The work cycle includes the following steps:
- opening of the loading door (3),
- loading of the objects to be perfumed (P) by placing them on the first jaw (71),
- tightening of the jaws (71, 72) by operating the handle (90),
- closing of the loading door (3),
- operating the start button (17) which starts the electric motor (6) at the preset number of revolutions and the pump (52) to send the perfumed liquid to the spary nozzles (4) for the preset nebulization time,
- perfuming cycle for the preset time,
- suction of air into the misting chamber (15) by means of the fan (200) at the end of the perfuming cycle,
- automatic unlocking of the electric lock (35) of the loading door (3) at the end of the nebulization and air suction cycle,
- opening of the gripping means (7) by manually operating the handle (90), and
- unloading of the products (P) scented by the gripping means (7).

To the present embodiment of the invention, equivalent variations and modifications can be made, within the reach of a skilled man, which however fall within the scope of the invention expressed by the attached claims.

## Claims

1. Scenting machine (100) for objects (P) to be scented, comprising:
- a frame (1),
- a misting chamber (15) inside the frame,
- a load door (3) suitable for opening/closing an opening (10) of the frame (1) that provides access to the misting chamber (15),
- spray nozzles (4) arranged in the misting chamber,
- a container (5) suitable for being filled with a scented liquid (50),
- a hydraulic conduit (51) connecting the container (5) to the spray nozzles (4),
- a pump (52) arranged in the hydraulic conduit (51) to feed the scented liquid to the spray nozzles (4),
- a flywheel (V) mounted in the misting chamber (15) and rotating around an axis of rotation (X) that extends longitudinally,
- an electric motor (6) that drives said flywheel (V) in rotation, and
- holding means (7) connected to said flywheel (V) and suitable for holding said objects (P) to be scented;
**characterized in that**
said holding means (7) comprises a holder comprising a first jaw (71) and a second jaw (72) that hold the objects (P) to be scented;
wherein the first jaw (71) is in the shape of a plate extending from the flywheel (V) into the misting chamber (15) in a direction parallel to the axis of rotation (X), and the second jaw (72) is in the shape of a rod extending from the flywheel (V) into the misting chamber (15) in a direction parallel to the axis of rotation (X) and diametrically opposite to the first jaw (71).

2. The scenting machine (100) according to claim 1, wherein said spray nozzles (4) are mounted in an upper part and in a rear part of the frame (1) and are oriented inferiorly and towards the center of the misting chamber (15).

3. The scenting machine (100) according to claim 2, wherein each one of said spray nozzles is oriented downwardly at an angle (α) of 30°-60° with respect to a horizontal plane.

4. The scenting machine (100) according to any one of the preceding claims, comprising drive means (9) that actuate the holding means (7) to hold the objects (P) to be scented between the holding means or to release the objects from the holding means.

5. The scenting machine (100) according to claim 4, wherein said flywheel (V) comprises a front disk (2) facing the misting chamber and a rear disk (8) disposed coaxially behind the front disk; the rear disk (8) being connected to a shaft (62) driven into rotation by the electric motor (6) about the axis of rotation (X); said front disk (2) being rotatable with respect to the rear disk (8); said drive means (9) being capable of enabling and disabling the rotation of the front disc (2) with respect to the rear disc (8); said first and second jaws (71, 72) of the holding means having followers (73, 74) mounted in slots (21, 81; 22, 82) of the front disc and the rear disc, in such a way that a rotation of the front disc with respect to the rear disc causes an approaching or receding movement of the jaws (71, 72) of the holding means.

6. The scenting machine (100) according to claim 5, wherein a first rectilinear slot (81) and a second rectilinear slot (82) arranged in diametrically opposite positions with respect to the center of the rear disc are formed in the rear disc (8); a first curved slot (21) shaped like a comma, and a second curved slot (82) shaped like a comma, arranged on either side with respect to the center of the front disc are formed in the front disc (2); the follower (73) of the first jaw (71) of the holding means (7) engages with the first curved slot (21) of the front disc and the first rectilinear slot (81) of the rear disc, and the follower (74) of the second jaw (72) of the holding means (7) engages with the second curved slot (22) of the front disc and the second rectilinear slot (82) of the rear disc.

7. The scenting machine (100) according to claim 5 or 6, wherein the drive means (9) comprise:
- a handle (90) protruding from the front disc (2) at a peripheral position in such a way to be grabbed by a user so that the user can rotate the front disc (2) relative to the rear disc (8), and
- a locking device (91) mounted in the front disc (2) and protruding rearward from the front disc (2) to lock the rear disc (8) to the front disc.

8. The scenting machine (100) according to claim 7, wherein the rear disk (8) is a gear wheel having a toothing (85) and the locking device (91) is a ratchet engaging with the toothing (85) of the gear wheel to lock the front disk to the rear disk.

9. The scenting machine (100) according to any one of the preceding claims, comprising a fan (200) for sucking the air into the misting chamber (15); said fan (200) being connected to an exhaust duct (203) having a first outlet (204) for discharging a gaseous component to the outside through a filter (206) and a second outlet (205) for discharging a liquid/solid component into a discharge container (207).
